# EUROPÄISCHE PATENTANMELDUNG

(11) **EP 1 281 354 A2**
(43) Veröffentlichungstag der Anmeldung: **05.02.2003**
(21) Anmeldenummer: 02017212.8
(22) Anmeldetag: 31.07.2002
(51) Int. Cl.: A61B 8/13, A61B 8/08

(54) **Verfahren und Applikator für die Spiral-Computer-Tomographie mit Ultraschall in der Medizin**

(30) Priorität: 31.07.2001 DE 10137186
(71) Anmelder: Ermert, Helmut, Prof. Dr.-Ing., 91341 Röttenbach (DE); Ashfaq, Mohammad, Dipl.-Ing., 44801 Bochum (DE); Hiltawsky, Karsten, Dr.med. Dipl.-Ing., 58239 Schwerte (DE)
(72) Erfinder: Ermert, Helmut, Prof. Dr.-Ing., 91341 Röttenbach (DE); Ashfaq, Mohammad, Dipl.-Ing., 44801 Bochum (DE); Hiltawsky, Karsten, Dr.med. Dipl.-Ing., 58239 Schwerte (DE)

(57) **Zusammenfassung**

Es ist bekannt, daß sich gutartiges von bösartigem Brustgewebe mit Hilfe der beiden akustischen Parameter Dämpfung und Schallgeschwindigkeit unterscheiden läßt. Das hier vorgeschlagene Verfahren soll in Verbindung mit dem vorgestellten Applikator dazu dienen, die dreidimensionale tomographische Rekonstruktion der beiden genannten akustischen Parameter im Zusammenspiel mit handelsüblichen Ultraschallgeräten zu ermöglichen.

Der Einsatz konventioneller Ultraschallgeräte wird dadurch ermöglicht, daß der Ultraschallwandler in einer Halterung angebracht wird, die starr mit einem gegenüberliegenden Reflektor verbunden ist. Beschreibt diese Halterung nun während des Meßvorgangs eine spiralförmige Bahn um das Objekt, können Meßdaten des gesamten Objektes in Transmissionsrichtung für verschiedene Projektionswinkel aufgenommen werden. Mit Hilfe von Rekonstruktionsverfahren ist nun eine dreidimensionale Darstellung der gewünschten Parameter mit hoher Ortsauflösung möglich.

Verfahren und Applikator sind für die Diagnostik von Brustgewebe konzipiert, können aber auch für die Untersuchung anderer Organe und Körperteile von Interesse sein, bei denen die anatomischen Gegebenheiten die Anwendung erlauben (z.B. Hoden).

## Beschreibung

Die Erfindung betrifft ein Verfahren zur dreidimensionalen Abbildung medizinischer Objekte mittels Spiral-Computer-Tomographie mit Ultraschall entsprechend dem Oberbegriff des Anspruchs 1 und einen Applikator entsprechend dem Oberbegriff des Anspruchs 6.

### Anwendungsgebiet

In den westlichen Industrienationen erkrankt gegenwärtig jede neunte Frau im Laufe ihres Lebens an einem bösartigen Brusttumor. Damit ist das Mammakarzinom die häufigste bösartige Tumorerkrankung des weiblichen Geschlechts. Als einziges Screeningverfahren zur Früherkennung hat sich bisher die Röntgen-Mammographie etabliert. Bei Verdacht auf einen Brusttumor schließt sich in den meisten Fällen zur Sicherung der Diagnose eine Ultraschalluntersuchung an. Allerdings kann auch mit Hilfe von konventionellen Ultraschallverfahren (B-Mode-Verfahren) nicht immer eine eindeutige Aussage über die Dignität (Gut- bzw. Bösartigkeit) des Brustgewebes getroffen werden. Zum einen handelt es sich um qualitative Abbildungsverfahren, deren Interpretation von der Erfahrung des Untersuchers abhängt, zum anderen ist die Untersuchung selbst von der Geschicklichkeit des Untersuchers abhängig, so dass Ergebnisse häufig nicht vergleichbar sind.
Es ist bekannt, dass die Ausbreitung von Ultraschall in Gewebe in Abhängigkeit von dessen akustischer Dämpfung und Schallgeschwindigkeit erfolgt. In *in-vitro* Studien stellte sich heraus, dass sich gutartiges von bösartigem Brustgewebe bzgl. dieser beiden physikalischen Parameter signifikant unterscheidet [1,2]. In klinischen Studien bestätigte sich, dass die ortsaufgelöste Darstellung der beiden quantitativen Gewebeparameter Dämpfung und Schallgeschwindigkeit entscheidend zur Differentialdiagnose von Brusttumoren beiträgt [3,4]. Das hier vorgeschlagene Verfahren in Verbindung mit dem hier vorgestellten Applikator soll dazu dienen, die dreidimensionale tomographische Rekonstruktion dieser beiden Gewebeparameter im Zusammenspiel mit handelsüblichen Ultraschallgeräten jeder Art für die Mammadiagnostik zu ermöglichen. Verfahren und Applikator können auch für die Untersuchung anderer Organe und Körperteile, bei denen die anatomischen Gegebenheiten die Anwendung erlauben, von Interesse sein (z.B. Hoden).

### Stand der Technik

Die Abbildung der Gewebeparameter Schallgeschwindigkeit und Dämpfung ist bisher nicht in konventionellen Ultraschallgeräten implementiert. Die Schätzung von Dämpfung und Schallgeschwindigkeit direkt aus Echodaten (aus dem Gewebe) ist zwar möglich, aber sehr ungenau und an bestimmte Bedingungen gebunden. Die genaue, ortsaufgelöste Rekonstruktion und Darstellung dieser akustischen Parameter ist besser mit tomographischen Konzepten aus verschiedenen Projektionen eines Objektes unter Nutzung von Transmissionsmessdaten möglich. Handelsübliche Ultraschallgeräte arbeiten aber im Reflexionsmodus und sind mit ihrer regulären Betriebsart nicht dazu geeignet.

Bisher wurden zwei Vorgehensweisen verfolgt, um Ultraschalldaten eines Objektes für verschiedene Projektionswinkel aufzunehmen und im Anschluß daran aus diesen Daten die genannten akustischen Parameter zu rekonstruieren.
Um die erforderlichen Messdaten im ***Transmissionsbetrieb*** zu erfassen, wurden spezielle Ultraschallsysteme entwickelt, die im wesentlichen aus zwei sich gegenüberliegenden Ultraschallwandlern oder Wandlersystemen ("Gruppenwandler" bzw. Arrays) bestehen (z.B. [7]). Diese Ultraschallwandler sind mechanisch starr miteinander gekoppelt und können sich um das Objekt drehen. Während der eine Ultraschallwandler Ultraschallwellen aussendet, kann der gegenüberliegende Ultraschallwandler die sich durch das dazwischen liegende Objekt ausbreitenden Schallwellen empfangen. Mit Hilfe verschiedener Drehwinkel kann für eine Schicht des Objektes ein Datensatz für beliebig viele Projektionen gewonnen werden.
Um Informationen über die Schallgeschwindigkeit und die Schalldämpfung im ***Reflexionsbetrieb*** zu erfassen, wurde mit einem Hilfsreflektor gearbeitet, der sich hinter dem Objekt, also auf der dem Echosonographie-Wandler abgewandten Seite, befindet. Mit dieser Anordnung durchlaufen die Ultraschallsignale das Objekt zweimal und gelangen als Echos vom Hilfsreflektor zum Wandler zurück. Dieses Konzept wurde in der Mammadiagnostik angewandt: dazu wurde eine Anordnung mit zwei horizontalen Platten benutzt, wobei das Objekt (hier die weibliche Brust) - ähnlich wie in einem konventionellen Mammographie-System - zwischen den Platten komprimiert wird [4]. Während die obere Platte aus Kunststoff angefertigt wird, um die Ausbreitung von Schallwellen von einem darüber angebrachten Schallwandler zu gewährleisten, besteht die untere Platte aus Metall und dient als Reflektor für die Schallwellen, die demnach das zu untersuchende Objekt zweimal durchlaufen, bevor sie von dem über der Kunststoffplatte liegenden Schallwandler wieder empfangen werden können.

### Nachteile des Standes der Technik

Alle Ansätze, bei denen die Erfassung der Messdaten im ***Transmissionsbetrieb*** erfolgt, sind sowohl von einem speziellen mechanischen Aufbau als auch von speziell entwickelten und miteinander mechanisch fixierten Ultraschallwandlern abhängig. Außerdem konnten die akustischen Parameter Schallgeschwindigkeit und Dämpfung bisher nur zweidimensional und mit einer eingeschränkten Ortsauflösung rekonstruiert werden. Die eingeschränkte Ortsauflösung ist unter anderem dadurch zu erklären, dass mit speziell anzufertigenden Ultraschallwandlern aus Sende- und Empfangselementen keine ausreichend fokussierten Ultraschallwellen erzeugt werden können.

Bei der Erfassung der Messdaten im ***Reflexionsbetrieb*** sind Einschränkungen bzgl. der Einsatzfähigkeit des Kompressionsapparates im klinischen Routinebetrieb bei der Mammadiagnostik genauso bekannt [6] wie eine stark eingeschränkte Ortsauflösung [5]. Letztere wird im Gegensatz zu Systemen im *Transmissionsbetrieb* aber nicht durch unzureichend fokussierbare Ultraschallwellen, sondern durch eine begrenzte Komprimierbarkeit des Objektes hervorgerufen, die wiederum den maximal möglichen Bereich der für eine Rekonstruktion erforderlichen Projektionswinkel entscheidend limitiert.

Insgesamt kann man sagen, dass alle bisher entwickelten Systeme im klinischen Routinebetrieb gescheitert sind, weil sie entweder in der Handhabung zu kompliziert oder in der Ortsauflösung unzureichend waren.

### Aufgabe der Erfindung

Die Erfindung dient dazu, die für eine dreidimensionale Abbildung der Gewebeparameter Schallgeschwindigkeit und Dämpfung notwendigen Messdaten mittels eines spiral-tomographischen Verfahrens unter Nutzung eines handelsüblichen Ultraschallgeräts zu erfassen, dabei gleichzeitig eine Ortsauflösung zu garantieren, die eine aussichtsreiche klinische Anwendung ermöglicht, und außerdem die simultane Nutzung des Ultraschallgerätes in seiner üblichen Betriebsart zur Aufnahme echosonographischer Bilder des Objektes (z.B. B-Bild, Doppler-Bild etc.) zu gestatten.

### Lösung der Aufgabe

Diese Aufgabe wird durch ein Verfahren mit den Merkmalen nach Anspruch 1 und einen Applikator mit den Merkmalen nach Anspruch 6 gelöst.

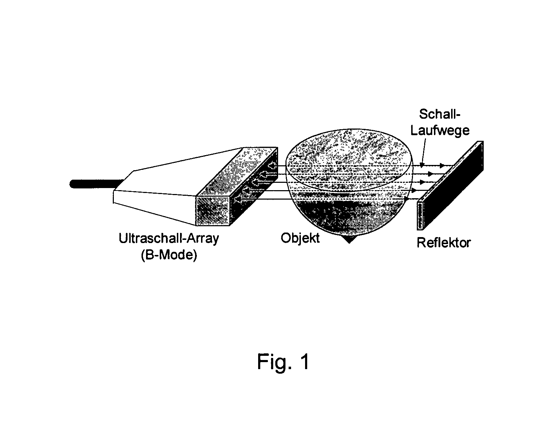

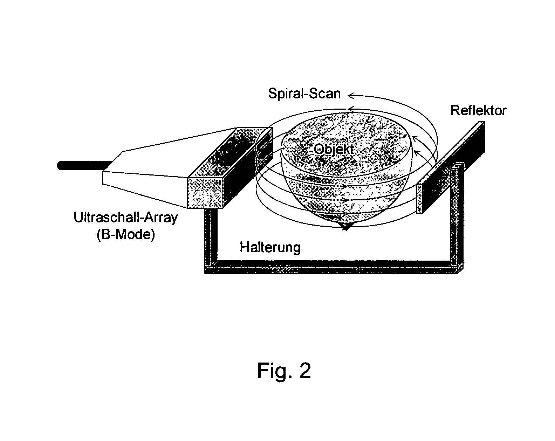

Das von uns vorgeschlagene Verfahren nutzt ein handelsübliches Ultraschallgerät, einen speziellen mechanischen Applikator und einen PC. Die Konfiguration und die Funktionsweise des Applikators sind in Zeichnungen Fig. 1 und Fig. 2 schematisch veranschaulicht.

Der Applikator besteht im wesentlichen aus einer Halterung für einen handelsüblichen Schallwandler und einem gegenüberstehenden Metallreflektor, der eine synchrone Drehbewegung mit der Halterung bei gleichzeitigem Vorschub senkrecht zur Drehebene ausführen kann. Dazu wird die Halterung für den Ultraschallwandler an ein zylindrisches Becken montiert, das gleichzeitig für die Dauer der Untersuchung mit Wasser gefüllt werden kann, um eine ausreichende akustische Ankopplung zu gewährleisten. Der metallische Reflektor wird so in das zylindrische Becken eingebracht, dass er dem eingespannten Ultraschallwandler gegenüber liegt. Die Ultraschallsignale durchlaufen das Objekt zweimal, bevor sie zum Wandler zurück gelangen (siehe Fig. 1). Die Drehbewegung der Halterung bzw. des Reflektors bei gleichzeitigem Vorschub senkrecht zur Drehbewegung wird mit Hilfe zweier Schrittmotoren realisiert. Der eine Schrittmotor wird sowohl mit dem zylindrischen Becken als auch mit einer Hubvorrichtung starr verbunden, um eine gleichmäßige Drehbewegung zu gewährleisten. Der andere Schrittmotor ist mit einem Gewinde verbunden, an das die Hubvorrichtung angebracht ist, so dass an dem zylindrischen Becken ein beliebiger Vorschub vorgenommen werden kann. Bei gleichzeitigem Betrieb beider Motoren führt ein in der Halterung eingespannter Ultraschallwandler eine schraubenförmige (im Sprachgebrauch "spiralförmige") Bewegung um ein von oben in das wassergefüllte Becken reichendes Objekt (weibliche Brust). Dieser Vorgang ist in Zeichnung Fig. 2 skizziert.

Die Datenaufnahme erfolgt abgestimmt auf die spiralförmige Bewegung des Ultraschallwandlers, so dass im Anschluß mit gängigen Rekonstruktionsverfahren (wie der "gefilterten Rückprojektion") die akustischen Parameter Schallgeschwindigkeit und Dämpfung rekonstruiert werden können.

Falls der Durchmesser des Objektes oder des darzustellenden Objektbereiches größer ist als die Wandlerbreite bzw. die Breite des echosonographischen Einzelbildes, kann ein zusätzlicher lateraler Schwenkvorgang der Wandler-Reflektorkombination in den Spiral-Scan-Vorgang integriert werden.

Alternativ kann anstelle der Metallplatte ein angepasster Ultraschallwandler mit entsprechender Elektronik treten. Falls (Alternative a) damit ein Empfangsbetrieb realisiert wird, müssen die gesendeten und fokussierten Ultraschallwellen das Objekt nicht zweimal passieren. Für die Datenerfassung kann eine Empfangseinheit außerhalb des Ultraschallgerätes eingesetzt werden. Eine weitere Alternative (b) ist die Nutzung dieses Wandlers im Sinne eines autonomen Senders oder eines Transponders nach Art des Sekundär-Radars. Beide Alternativen haben den Vorteil größerer effektiver Reichweite bzw. der Anwendung höherer Frequenzen.

Es gibt zwei besondere Probleme: I) die Breiten der handelsüblichen Schallwandler-Arrays können kleiner sein als die Durchmesser der zu untersuchenden Organe, II) auch Schnittebenen möglichst nah an der Thoraxwand der Patientinnen sollen erfasst werden können. Zu I: die Transmissions-CT erfordert nur einen 180°-Scan, die Spiral-CT "dreht" aber kontinuierlich über 360°. Hier können Redundanzen genutzt werden. Z.B. lassen sich eine "Aufwärtsspirale" mit halbseitig versetztem Wandler und eine Abwärtsspirale mit zur anderen Seite versetztem Wandler fahren. Entsprechende Optionen sind bei den Konstruktionen zu nutzen. Ein weiteres Beispiel ist die Möglichkeit der Nutzung von Convex-Arrays in Verbindung mit gekrümmten Reflektoren. Zu II: Anders als in den Zeichnungen Fig. 1 und Fig. 2 dargestellt, kann mittels eines totalreflektierenden Spiegels der Schall von einem seitlich schräg von unten sendenden Wandler sehr dicht unter die Wasseroberfläche gebracht werden. Dieses Konzept wurde bereist erfolgreich bei einem Hodenscanner (Ultraschall-Echo-CT, [8]) erprobt, für den ähnliche Anforderungen bestanden.

### Vorteile der Erfindung

Durch die mit diesem Applikator mögliche Spiralbewegung können Messdaten für ein Objekt so erfasst werden, dass eine dreidimensionale Rekonstruktion der akustischen Parameter Schallgeschwindigkeit und Dämpfung mit hoher Ortsauflösung möglich wird. Die Halterung für Ultraschallwandler wurde so konzipiert, dass handelsübliche Wandlertypen, die in ihrer Form nicht wesentlich voneinander abweichen, in ihr fixiert werden können. Mit Hilfe einer Metallplatte als Reflektor können die Ultraschallwandler im konventionellen Reflexionsbetrieb (B-Modus) betrieben werden, so dass stark fokussierte Ultraschallwellen sowohl gesendet als auch empfangen werden können.

Als weiterer Vorteil ist zu sehen, dass die Erfassung des gesamten Objektes untersucherunabhängig verläuft und die extrahierten (quantitativen) Parameter ein objektives und reproduzierbares Maß in der Gewebecharakterisierung darstellen. Vergleichbar mit anderen dreidimensionalen Abbildungsverfahren (z.B Magnet-Resonanz-Tomographie) können darüber hinaus aus den dreidimensionalen Parameter-Datensätzen beliebige Schnittebenen rekonstruiert werden.

Ein weiterer Vorteil ist darin zu sehen, dass nicht nur ein handelsübliches Ultraschallgerät genutzt werden kann, das sich bezüglich Wandlertechnologie, Strahlformung, Signal- und Bildverarbeitung auf einem hohen Entwicklungsstand befindet, sondern dass simultan die konventionellen echosonographischen Abbildungsmodalitäten ausgewertet und mit den Spiral-CT-Ergebnissen kombiniert werden können.

### Literaturangaben:

[1] F.S. Foster, M. Strban, G. Austin. The ultrasound macroscope: Initial studies of breast tissue. *Ultrasonic Imaging,* 6:243-261, 1984.
[2] P.D. Edmonds, C.L. Mortensen, J.R. Hill, S.K. Holland, J.F. Jensen, A.D. Valdes. Ultrasound tissue characterization of breast biopsy specimens. *Ultrasonic Imaging,* 13(2): 162-185, 1991.
[3] C.L. Mortensen, P.D. Edmonds, Y. Gorfu, J.R. Hill, J.F. Jensen, P. Schattner, L.A. Shifrin, A.D. Valdes, S.S.J. and L.J. Esserman. Ultrasound tissue characterization of breast biopsy specimen: Expanded study. *Ultrasonic Imaging,* 18(3):215-230, 1996.
[4] K. Richter, Clinical amplitude/velocity reconstructive imaging (CARI) - a new sonographic method for detecting breast lesions. *Brit. J. Radiol.,* 68:375-384, 1995.
[5] M. Krueger, V. Burow, K. M. Hiltawsky, H. Ermert. Limited angle ultrasonic transmission tomography of the compressed female breast. 1998 *IEEE Ultrasonics Symposium.* Proceedings (Cat. No. 98CH36102). IEEE. Part vol.2, 1998, pp.1345-8 vol.2. Piscataway, NJ, USA.
[6] K. M. Hiltawsky, M. Krüger, C. Starke, A. Jensen, L. Heuser, H. Ermert. Freehand Elastography of breast lesions: Clinical results. *Ultrasound in Med. & Biol.,* in press.
[7] J. F. Greenleaf: Computerized Tomography with Ultrasound. *Proc. IEEE*, vol. 71, pp. 330 - 337, Mar. 1983.
[8] G. Röhrlein: Computer-Tomographie mit Ultraschall-Echosignalen für die medizinische Diagnostik. Dissertation Universität Erlangen-Nürnberg (Technische Fakultät) 1986.

### B. Beispielbeschreibung einer Patentanmeldung

Schematische Zeichnungen sowohl des Applikators als auch des Scan-Vorgangs sind in den Zeichnungen Fig. 1 bzw. Fig. 2 zu finden. Dabei ist insbesondere die Anordnung aus Ultraschall-Array und metallischem Reflektor dargestellt, die eine spiralförmige Bewegung um das Objekt beschreibt (hier: weibliche Brust).

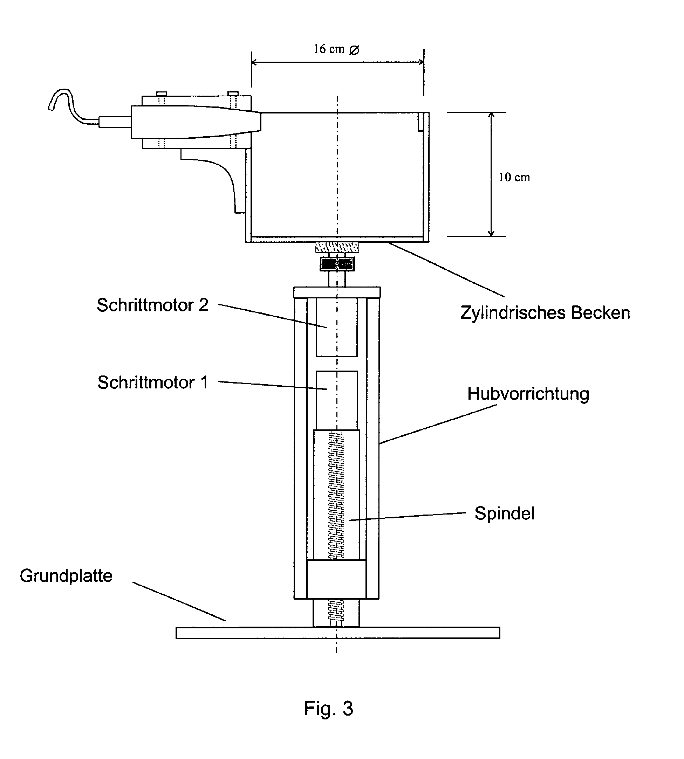

Ausführungszeichnungen der Erfindung sind in den Zeichnungen Fig. 3, Fig. 4 und Fig. 5 dargestellt und werden im folgenden näher beschrieben. Der Applikator besteht aus folgenden Funktionseinheiten:

### Zu Fig. 3: Ansicht des Applikators

a) Eine Grundplatte, auf der eine drehbare Spindel mit Schrittmotor (Schrittmotor 1) befestigt ist.
b) Eine Hubvorrichtung, an der ein zweiter Schrittmotor befestigt ist (Schrittmotor 2), mit dessen Hilfe ein gelagertes zylindrisches Becken eine Drehbewegung ausführen kann. Die Hubvorrichtung kann mit Hilfe des unter a) genannten Schrittmotors (Schrittmotor 1) senkrecht zur Drehebene des zylindrischen Beckens beliebig bewegt werden.
c) Das während einer Untersuchung wassergefüllte zylindrische Becken dient nicht zur Unterbringung des Objektes, sondern garantiert außerdem, dass die unter d) genannte Halterung bzw. der unter e) genannte metallische Reflektor eine synchrone spiralförmige Bewegung um das Objekt ausführen.

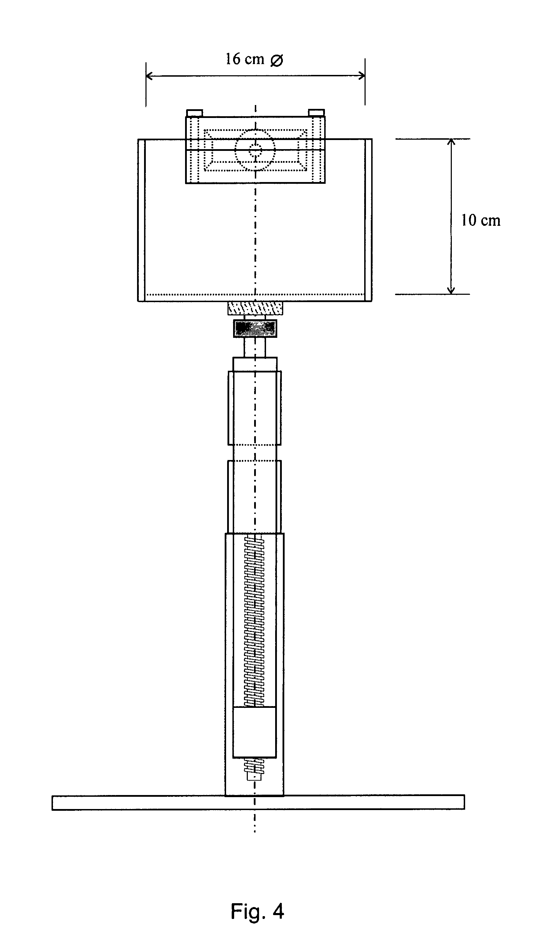

### Zu Fig. 4: Seitenansicht des Applikators

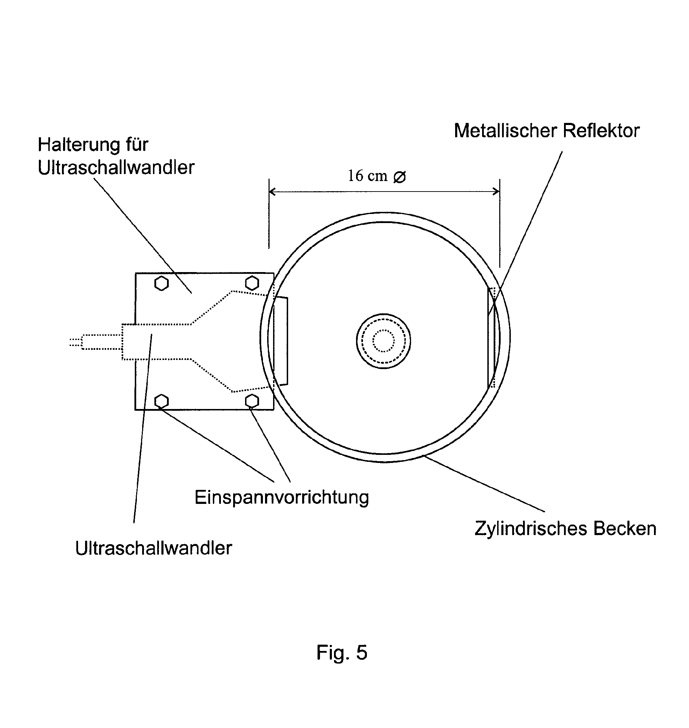

### Zu Fig. 5: Draufsicht des Applikators

d) Eine an das zylindrische Becken angebrachte Halterung für Ultraschallwandler mit Einspannvorrichtung, um handelsübliche Modelle fixieren zu können.
e) Ein Metallreflektor aus rostfreiem Edelstahl, der eine Reflexion der fokussierten Ultraschallwellen gewährleistet. Das zu untersuchende Objekt befindet sich während eines Messvorgangs im mit Wasser gefüllten zylindrischen Becken zwischen diesem metallischen Reflektor und dem eingespannten Ultraschallwandler.

## Patentansprüche

1. Ein Verfahren zur dreidimensionalen Abbildung von Objekten mittels Ultraschall, **dadurch gekennzeichnet, dass** das betreffende Objekt von einem Ultraschall-Sensorsystem zur Aufnahme sonographischer Messdaten entsprechend dem in der Röntgendiagnostik üblichen Verfahren der Spiral-Computer-Tomographie auf einer Schraubenlinie umkreist wird und dabei die dreidimensionale Objektinformation gewonnen wird.

2. Ein Verfahren nach Anspruch 1, **dadurch gekennzeichnet, dass** das Ultraschall-Sensorsystem echosonographische Messdaten erfasst.

3. Ein Verfahren nach Anspruch 1, **dadurch gekennzeichnet, dass** das Ultraschall-Sensorsystem transmissionssonographische Messdaten erfasst.

4. Ein Verfahren nach Anspruch 1, **dadurch gekennzeichnet, dass** das Ultraschall-Sensorsystem echosonographische und transmissionssonographische Messdaten erfasst.

5. Ein Verfahren nach Anspruch 3 oder 4, **dadurch gekennzeichnet, dass** das Ultraschall-Sensorsystem aus einem Ultraschallwandler, der im Puls-Echo-Betrieb arbeitet, und einem Hilfsreflektor, der auf der dem Ultraschallwandler abgewandten Seite des Objektes angebracht und über eine Halterung mit dem Wandler starr verbunden ist, besteht, wobei Transmissionssignale mittels Puls-Echo-Messung dadurch gewonnen werden, dass die Echosignale vom Hilfsreflektor hinter dem Objekt als Transmissionssignale ausgewertet werden, die das Objekt zwei mal durchlaufen haben.

6. Ein Applikator für die Spiral-Computer-Tomographie der weiblichen Brust oder anderer Organe mit Ultraschall nach Anspruch 5, **dadurch gekennzeichnet, dass** ein beliebiger Ultraschallwandler befestigt werden und im konventionellen Reflexionsbetrieb Schallwellen dadurch empfangen kann, dass auf der gegenüberliegenden Seite als Hilfsreflektor eine Metallplatte installiert ist.

7. Ein Applikator nach Anspruch 5, **dadurch gekennzeichnet, dass** die Halterung des Ultraschallwandlers synchron mit der gegenüberliegenden Metallplatte Drehbewegungen ausüben kann bei *gleichzeitigem* Vorschub dieser Anordnung senkrecht zur Drehebene, wodurch eine spiralförmige Bahn um ein Objekt herum durchlaufen werden kann.

8. Ein Applikator nach Anspruch 5, **dadurch gekennzeichnet, dass** die synchrone Bewegung der Halterung für den Ultraschallwandler bzw. des metallischen Reflektors durch eine mechanische Fixierung an ein zylindrisches Becken realisiert wird, das außerdem mit Wasser gefüllt werden kann, um eine ausreichende akustische Ankopplung des zu untersuchenden Objektes für die Dauer des Messvorgangs zu gewährleisten.

9. Ein Applikator nach einem der Ansprüche 5 bis 8, **dadurch gekennzeichnet, dass** simultan zur Aufnahme transmissionssonographischer Daten auch echosonographische Daten aufgenommen und verarbeitet werden können.

10. Ein Verfahren nach Anspruch 5, **dadurch gekennzeichnet, dass** anstelle eines Hilfsreflektors ein Ultraschallwandler verwendet wird, der die Transmissionssignale zur Weiterverarbeitung im Sinne der Transmissions-Computer-Tomographie aufnimmt.

11. Ein Verfahren nach Anspruch 5, **dadurch gekennzeichnet, dass** anstelle eines Hilfsreflektors ein Ultraschallwandler verwendet wird, der die Transmissionssignale aufnimmt und verstärkt oder codiert oder verstärkt und kodiert zurücksendet.
